# EUROPEAN PATENT APPLICATION

(11) **EP 0 521 464 A1**
(43) Date of publication of application: **07.01.1993**
(21) Application number: 92111065.6
(22) Date of filing: 30.06.1992
(51) Int. Cl.: C07D 241/46, C12P 17/12, C12R 1/465, A61K 31/495, C12N 1/20

(54) **Antibiotic, phencomycin, a process for its production and its use**

(30) Priority: 03.07.1991 EP 91111040
(71) Applicant: HOECHST AKTIENGESELLSCHAFT, 65926 Frankfurt am Main (DE)
(72) Inventor: Franco, Christopher Milton Mathew, Dr., Bombay 400 022 (IN); Maurya, Rajkumar, Bombay 400 066 (IN); Chatterjee, Sugata, Dr., Bombay 400 082 (IN); Ganguli, Bimal Naresh, Dr., Chembur, Bombay 400 971 (IN); Blumbach, Jürgen, Dr., W-6272 Niedernhausen (DE)

(57) **Abstract**

Phencomycin, a compoud of the formula 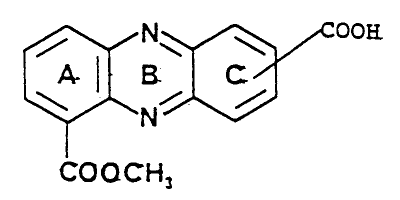
has an antibiotic action, an action as enzyme inhibitor and an influence on the kallikrein-kinin system.

## Description

This invention refers to Phencomycin, a process for its production and its use.

As is apparent in the following detailed description of the invention, Phencomycin the compound of formula I of this invention belongs to the phenazine class of antibiotics, but differs from all known members of this class such as Tubermycin (J. Antibiotics Ser. A 11, 264, 1958), 2,9 Dihydrophenazine-1-carboxylic acid and 1,8 Phenazinediol (J. Heterocyclic Chem. 6, 297, 1969), Lomofungin (Appl. Microbiol. 17, 755, 1969), Phenazinecarboxylic acid (J. Org. Chem. 41, 2101, 1976, Chem. Abstr. 88, 11570, 1978), Phenazinedicarboxylic acid (J. Heterocycl. Chem. 6, 297, 1969; J. Chem. Res. 1982, 362), Saphenamycins (J. Antibiotics 35, 1412, 1982), Phenacein (J. Antibiotics 37, 1308 & 1313, 1984), PD 116152 (J. Antibiotics 39, 192, 1986), DC-86-M (J. Antibiotics 39, 619 & 624, 1986), Saphenic acid derivatives (J. Antibiotics 41, 1542 & 1552, 1988) and Phenazinomycin (J. Antibiotics 42, 1037, 1989). A compilation of known phenazine compounds can also be found in the "CRC Handbook of Antibiotic Compounds, Vol. V: Heterocyclic Antibiotics" (J. Berdy, Ed.), CRC Press, Boca Raton, Florida, 1981, in the "Index of Antibiotics from Actinomycetes" (H. Umezawa, Ed.), Univ. Park Press, Baltimore, USA, on pages 255, 320, 321, 667 and 668 in Vol. I (1967), and on pages 721, 722 and 794 in Vol. II (1978), in Advances in Applied Microbiology 13, 267-282, 1970, and in "Handbook of Microbiology" Vol. III (A. I. Laskin & H.A. Lechevalier, Eds.), pp 329-332, CRC press inc., Cleveland, 1973. The structure of Phencomycin is depicted in the following formula wherein a carboxylic acid group is present in Ring C.

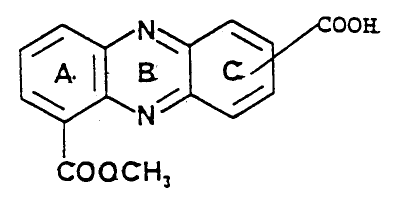

This invention furthermore provides a process for preparing the novel compound Phencomycin which comprises cultivating Str. sp. Y-90,31725, its variants and mutants, under aerobic conditions, at a temperature between 18°C and 40°C, in an aqueous nutrient medium containing sources of carbon, sources of nitrogen, and mineral salts, at a pH between 6 and 9 and recovering the antibiotic from the medium.

Str. sp. Y-90,31725 was isolated from soil collected at Bharatpur, Rajasthan, India. It has been deposited according to the conditions of the Treaty of Budapest with Deutsche Sammlung von Mikroorganismen, Braunschweig on April 30, 1991 (DSM 6520). Variants and mutants of culture number HIL Y-90,31725 can be obtained in a known manner using a mutagen, such as N-methyl-N-nitro-N'-nitrosoguanidine or ultraviolet light. The microorganism Str. sp. Y-90,31725 belongs to the order Actinomycetales, family Streptomycetaceae and genus Streptomyces.

Str. sp. Y-90,31725 is considered to be a new strain since it differs from the known strains in some of its morphological, cultural and physiological characteristics. It is considered to be a new strain also because it produces a new compound with antibiotic activity herein called Phencomycin as will be clear from the description hereinafter.

Phencomycin is active in vitro against a number of Gram-positive bacteria. Accordingly, it may be used as a therapeutic drug in human and veterinary medicine.

According to the present invention there is also provided a process for the isolation of Str. sp. Y-90,31725 from soil using a nutrient medium at a pH of 6.5 to 8.5 in known manner.

The nutrient medium used for isolation of the microorganism from soil consists of carbon and nitrogen sources, inorganic nutrient salts and solidifying agents. Sources of carbon may be glucose, starch, dextrin, glycerol, sucrose or molasses.

Sources of nitrogen may be peptone, yeast extract, beef extract, malt extract, casein or amino acids such as arginine or asparagine. The solidifying agent may, for example, be agar. The inorganic nutrient salts may be salts of sodium, potassium, magnesium, calcium, iron, zinc, manganese, copper, phosphorous or sulphur.

The microorganism of this invention eleongates aerial mycelia from branched substrate mycelia. Spores are formed in chains on top of aerial mycelia representative of Section Retinaculum Apertum. Neither whirl or ascospores are observed. Mature spore chains contain more than 30 spores per chain. The culture belongs to the Red series. Based on the cultural and morphological characteristics of the microorganism on various agar media, as well as cell wall analysis which shows the presence of LL-diaminopimelic acid, the producing organism can be identified as a Streptomyces species.

It may be well understood to those skilled in the art that this invention is not limited to the particular organism which has been specified above but includes all those spontaneous and artificial mutants and variants derived from the said microorganism which are capable of producing the new antibiotic Phencomycin.

According to the present invention there is also provided a process for the production of Phencomycin, said process comprising cultivating Str. sp. Y-90,31725 by fermentation at a pH between 6.0 and 9.0 and a temperature between 18-40°C under aerobic conditions in a nutrient medium containing sources of carbon and nitrogen, nutrient inorganic salts, and trace elements, and isolating the compound from the culture broth in a known manner such as herein described.

The carbon sources used in the nutrient medium for production of the novel compound may be e.g. glucose, starch, dextrin, glycerol, sucrose, molasses or oil. Sources of nitrogen used in the nutrient medium for production of the novel compound may be for instance soybean meal, yeast extract, beef extract, malt extract, cornsteep liquor, peptone, gelatin, casein, ammonium salts or nitrate salts.

Nutrient inorganic salts/mineral salts used in the nutrient medium for production of the novel compound may be sodium chloride, magnesium sulphate, ammonium sulphate, sodium nitrate, potassium nitrate or calcium carbonate. As trace elements, for instance iron, manganese, copper, zinc or cobalt may be used. If desired, an antifoaming agent such as Desmophen® (Polyols, Bayer AG, Leverkusen) may be used in the nutrient medium during fermentation of the culture.

Preferably, Str. sp Y-90,31725 is cultivated at about 27°C and pH about 7.0. The fermentation is stopped after 44-48 hours when maximum yields of the compound are obtained. The fermentation may, preferably, be submerged fermentation. The process of fermentation and formation of the novel antibiotic can be monitored by the antibacterial activity of the culture fluid and mycelium against Staphylococcus aureus 209 P in agar medium and by thin layer chromatography on silica gel plates with ethyl acetate as developing solvent.

Phencomycin can be obtained from the culture broth by extraction with a water-immiscible solvent after the pH has been adjusted to 4.5-6.5. The solvents could be ethyl acetate or chloroform; preferably, it is ethyl acetate and the preferred pH is 5.0. The solvent extract is concentrated to remove the solvent and then chromatographed further. Phencomycin can also be obtained from the culture broth by direct adsorption on suitable adsorbents such as Amberlite® XAD-4 or 7 (Rohm and Haas Co., U.S.A.), or Diaion® HP-20 (Mitsubishi Chemical Industries, Japan); the preferred adsorbent being Diaion® HP-20. The compound according to the invention is eluted from the adsorbent using appropriate mobile phases, such as methanol or acetone, either singly, in combination with each other, or with water, and the eluates are then evaporated to dryness. The preferred eluant is methanol. The active eluates thus obtained are pooled and concentrated.

The aforementioned concentrated eluates or extracts containing Phencomycin, can be further purified in a number of ways. For example, re-adsorption and elution processes with activated carbon, Amberlite® XAD-4 and 7. Diaion® HP-20; gel filtration with Sephadex® LH-20 gel (Pharmacia Fine Chemicals AB, Sweden) and its equivalents; adsorption chromatography on alumina and silica gel, can be conveniently combined for further purification. In addition, thin-layer chromatography, medium-pressure and high-pressure liquid chromatography using suitable adsorbents such as silica gel and modified silica gel-C₁₈ with suitable solvent systems may be used. Furthermore, counter current chromatography with a particular biphasic solvent system may work well for the said purpose. Preferably, silica gel chromatography with ethyl acetate and dichloromethane as the eluting solvent is used. Another purification process used either alone, or in combination with the above-mentioned purification procedures, is based on the differential solubility of Phencomycin in organic solvents and its pH-dependent solubility in aqueous solution. The aforementioned concentrated extracts or eluates containing Phencomycin may be precipitated with hexane, repeatedly, in known manner. Finally, Phencomycin may be crystallized in a suitable solvent, or mixture of solvents. The preferred solvent is dichloromethane.

Phencomycin is a yellow, crystalline powder. It is poorly soluble in acidic water, hexane and petroleum ether. It is fairly soluble in methanol and has a good solubility in acetonitrile, methylene chloride, ethyl acetate, chloroform, propylene glycol and dimethylsulfoxide.

The compound melts at 207°C.

In the thin-layer chromatography (using a precoated silica gel plate : Article No. 5554 from E. Merck, Darmstadt.) with ethylacetate as the developing solvent, Phencomycin has an Rf value of 0.43.

In the analytical high-pressure liquid chromatography (HPLC) carried out using the conditions mentioned below, the R_{T} = 11.63 min.

| | |
|---|---|
| Column packing: | ODS-Hypersil®-10 µ,4 x (30 + 250) mm |
| Flow Rate: | 2.0 ml/min |
| Detection: | 220 nm |
| Eluant: | gradient of DD H₂O to 100 % CHC₃N in 30 min. |

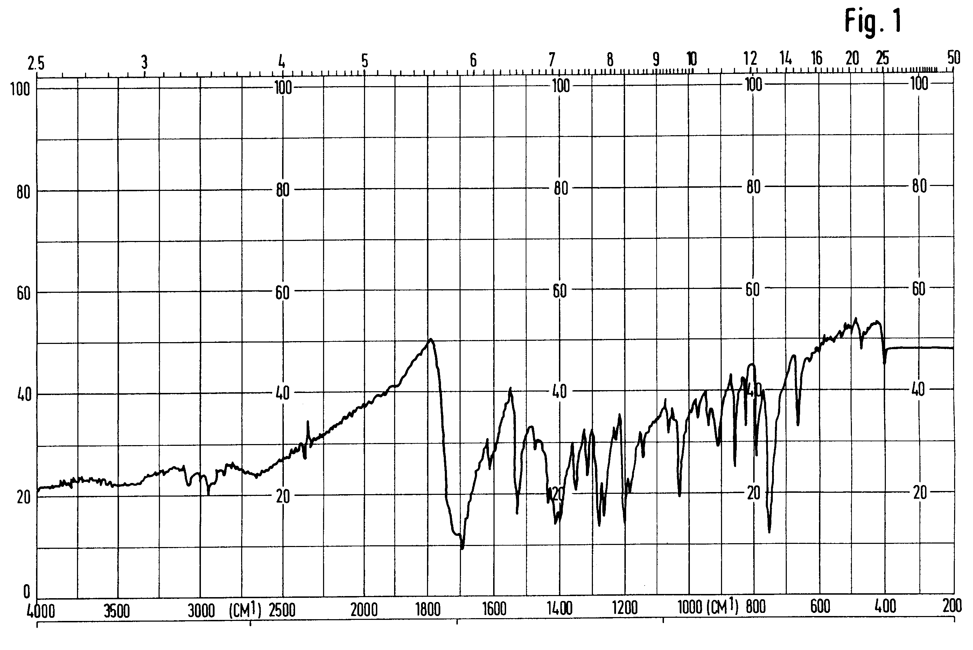

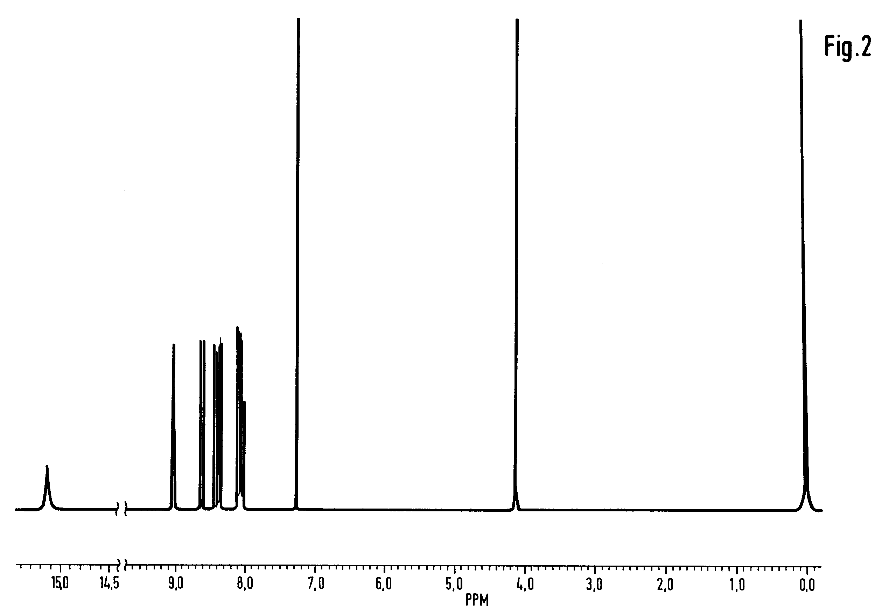

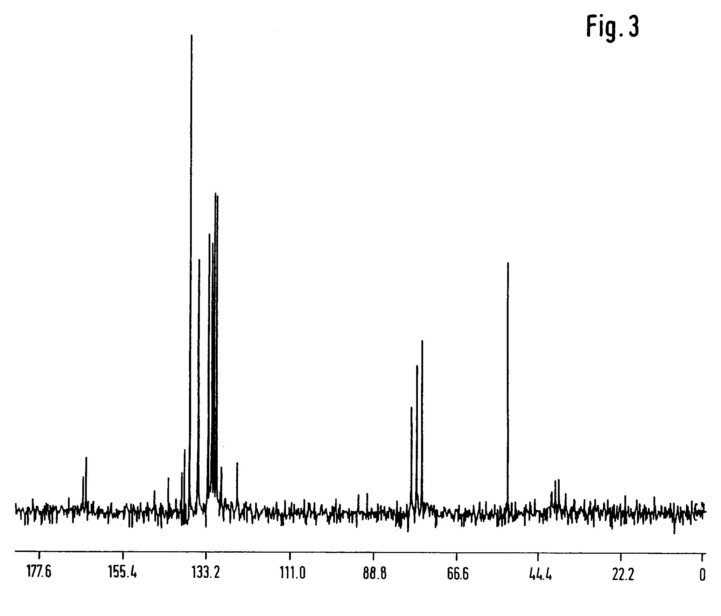

The spectroscopic data of Phencomycin is listed below:
1. UV λₘₐₓ in methanol is 254 & 368 nm; in 0.1 N HCl-methanol it is 250 & 371 nm; and in 0.1 N NaOH-methanol it is 258 & 366 nm. The absorption spectrum was measured in the range 200 to 800 nm using a Uvikon 810 Spectrophotometer.
2. The IR spectrum (KBr) was determined using a Perkin Elmer P.E. 521 IR Spectrometer - See Fig. 1 of the accompanying drawings.
3. The elemental analysis shows C = 63.83 %, H = 3.55 %, N = 9.91 %, O = 22.71 %.
4. The ¹H-NMR spectrum was determined on a 300 MHz BRUKER ACP-300 instrument using CDCl₃ as solvent - See Fig. 2 of the accompanying drawings.
5. The ¹³C-NMR spectrum was determined on a 90 MHz JEOL FX-90Q instrument using CDCl₃ as solvent - See Fig. 3 of the accompanying drawings.
6. Mass Spectroscopic analysis was carried out on a KRATOS MS 80 instrument using Desorption Chemical Ionization (DCI) and Electron Impact modes of ionization. The molecular weight was found to be 282. This in combination with the mass fragmention pattern and elemental analysis corresponds to a Molecular formula of C₁₅H₁₀N₂O₄.

The above data together with the chemical analysis indicates that Phencomycin is a novel compound with a structure as shown above wherein a carboxylic acid group is present in Ring C.

Phencomycin is active against gram-positive bacteria including clinical isolates such as Staphyloccus sp., Enterococcus sp., Mycobacterium sp. and anaerobic bacteria and may be used as an antibiotic compound.

In addition to the antibacterial activity, Phencomycin was found to have a pharmacological activity. It inhibits physiologically important enzymes such as renin and may therefore have a role in the treatment of cardiovascular disorders. It also interferes with the kallikrein-kinin system and hence may have a role in the control of certain pathological conditions like inflammation, asthma, pancreatitis and shock syndromes.

Accordingly the instant invention also relates to pharamaceuticals containing an active amount of Phencomycin and to the use of Phencomycin for the production of pharmaceuticals, in particular for pharmaceuticals having an antibiotic action or having an action as enzyme inhibitor or having an influence on the kallikrein-kinin system.

The invention will be further illustrated by preferred examples, but should not be considered as limited by those examples; the invention is furthermore characterized by the patent claims.

### Example I

### Isolation of Streptomyces sp. Y-90,31725 from soil

### (a) Preparation of nutrient isolation media

| | | |
|---|---|---|
| Composition: | Starch | 10.0 g |
| | Casein | 0.3 g |
| | KNO₃ | 2.0 g |
| | NaCl | 2.0 g |
| | K₂HPO₄ | 2.0 g |
| | MgSO₄·7H₂O | 0.05 g |
| | CaCO₃ | 0.02 g |
| | FeSO₄·7H₂O | 0.01 g |
| | Agar | 15.0 g |
| | Distilled water | 1 litre |
| | pH | 7.2-7.5 |

The media were sterilized at 121°C for 30 minutes. In all cases, the sterilized media were cooled to 45°C, poured into petri plates and allowed to solidify. Cycloheximide and sodium azide and were added to the cooled molten agar to give final concentrations of 25 µg/ml and 5 µg/ml respectively.

### (b) Preparation of soil suspension

On gram of soil was suspended in distilled water and shaken well. The soil was allowed to settle and the supernatant fluid was used to inoculate each one of the above-mentioned isolation media at a time.

### (c) Inoculation of the isolation medium

One ml of the soil suspension was inoculated onto petri dishes containing 50 ml of any of the above-mentioned nutrient isolation media.

### (d) Isolation of Streptomyces sp. Y-90,31725

The inoculated petri dish was incubated at 30°C for 10 days and Streptomyces sp. Y-90,31725 isolated from among the growing microorganisms.

### Example II

Cultivation of Streptomyces sp. Y-90,317255 for the fermentative production of Phencomycin Streptomyces sp. Y-90,31725 was maintained on yeast extract-malt extract having the following composition:

| | |
|---|---|
| Malt extract | 10.0 g |
| Yeast extract | 4.0 g |
| Glucose | 4.0 g |
| Agar | 15.0g |
| Distilled water | 1 litre |
| pH | 7.0 |

The medium was distributed in test tubes and sterilized at 121°C for 30 minutes. The tubes were cooled in a slanting position for preparation of agar slants. The slants were inoculated with the culture and incubated at 28°C for 10-15 days when good growth and sporulation were observed. A suspension of the spores in distilled water from one slant was used to inoculate five 500 ml Erlenmeyer flasks each containing 100 ml of the seed culture medium.

### Composition of the Medium A

| | |
|---|---|
| Glucose | 15.0 g |
| Soybean meal | 15.0 g |
| Cornsteep liquor | 5.0 g |
| CaCO₃ | 2.0 g |
| NaCl | 5.0 g |
| Distilled water | 1 litre |
| pH | 6.5 |

The above medium was distributed in 100 ml amounts in 500 ml Erlenmeyer flasks and sterilized at 121°C for 30 minutes.

The flasks were cooled, inoculated with spore suspension or mycelial plugs and shaken at 240 r.p.m. for 72 hours at 27°C (± 1°C) on a rotary shaker with 1.5 inch throw. The resultant growth was used to inoculate the 15 L fermenter containing 10 L of the Medium A at 9 % (v/v). 4 ml of Desmophen® was added as antifoam agent to the contents of the fermenter. The medium was sterilized through direct and indirect steam for 20 minutes at 121°C. The fermenter was cooled and inoculated with seed culture (9 % v/v). The fermentation was carried out at 27°C (± 1°C) under stirred conditions at 150 r.p.m. with aeration at a rate of 10 conditions at 150 r.p.m. with aeration at a rate of 10 liters per minute. When fermentation was discontinued at the end of 44-45 hours the pH of the culture broth was pH 7.88 and the diameter of the zone of inhibition, when tested by the agar well method, versus Staphylococcus aureus 209 P was 23 mm. The packed cell volume was 22 % (v/v). The culture broth was processed as in Example III.

### Example III

### Isolation and purification of Phencomycin

The culture filtrate (8 liters), from the fermentation as outlined in Example II, was passed through a 90 x 5 cm column containing 400 ml Diaion® HP-20 resin. The resin was then washed with water and eluted with a Distilled Water:Methanol stepwise gradient. The active eluates containing Phencomycin obtained with a Dist. Water:Methanol mixture of 5:5 to 2:8 were pooled and concentrated to 200 ml aqueous solution. This was extracted with 500 ml ethylacetate after adjusting the pH to 5. The ethylacetate layer containing Phencomycin was separated and then extracted with 200 ml of concentrated sodium bicarbonate solution. The bicarbonate solution was separated out, acidified using HCl to pH 5 and extracted with 500 ml ethylacetate. The extract was concentrated to dryness (850 mg) followed by chromatography on a 3 x 100 cm glass column containing 150 ml silica gel (200-300 mesh) set up in dichloromethane. This column was eluted with a dichloromethane:ethylacetate gradient with the active phencomycin eluting out when the ratio was 99.5:0.5 to 99:1. The active eluates were concentrated to dryness (150 mg) followed by crystallization in dichloromethane at -20°C. 100 mg fine yellow needle-like crystals were obtained.

## Claims

1. Phencomycin, a compound of the formula I

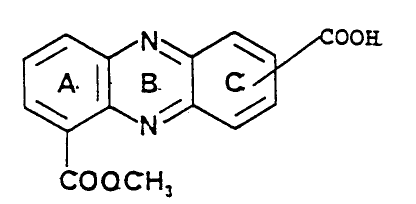

2. Phencomycin as claimed in claim 1, obtainable by cultivation of culture No. Y-90,31725 and isolating said compound of the fermentation broth and/or the mycelium.

3. A process for the production of Phencomycin as claimed in claims 1 or 2, wherein culture No. Y-90,31725 is cultivated in an aqueous nutrient medium containing sources of carbon, sources of nitrogen and mineral salts.

4. A process as claimed in claim 3, wherein cultivation is carried out at a temperature between 18 and 40°C and pH between 6 and 9.

5. A process as claimed in claims 3 or 4, wherein cultivation is carried out at a temperature about 27°C and pH about 7.0.

6. A pharmaceutical containing an active amount of Phencomycin as claimed in claim 1.

7. The use of Phencomycin as claimed in claim 1 for the production of a pharmaceutical.

8. The use of Phencomycin for the production of a pharmaceutical having an antibiotic action.

9. The use of Phencomycin as claimed in claim 1 for the production of a pharmaceutical having an influence on the kallikrein-kinin system or having an action as enzyme inhibitor.

10. Streptomyces species Y-90,31725 (DSM 6520).
